# EUROPEAN PATENT APPLICATION

(11) **EP 3 862 036 A1**
(43) Date of publication of application: **11.08.2021**
(21) Application number: 20305121.4
(22) Date of filing: 07.02.2020
(51) Int. Cl.: A61M 1/10, A61M 1/12

(54) **IMPLANTABLE DEVICE FOR LONG-TERM ASSIST OF THE RIGHT VENTRICLE OF A HEART**

(71) Applicant: HTC-Assistance, 92110 Clichy (FR)
(72) Inventor: MOUSSAFEUR, Amina, 92110 Clichy (FR)
(74) Representative: Icosa

(57) **Abstract**

The present invention relates to an implantable right ventricular assist device configured to be implanted within the right ventricle of a heart, comprising a percutaneously deliverable centrifugal pump 2 configured to carry the blood from the right ventricle to the pulmonary artery; a fixation device 3 configured to deliver and secure said centrifugal pump within a portion of the pulmonary artery; and a power source 4.

The invention also relates to a method to percutaneously and transluminally implant the Right Ventricular Assist Device 1 of the present invention within the right ventricle of a patient's heart.

The invention further relates to the use of the right ventricular assist device of the present invention to long-term assist of the right ventricle of a patient's heart.

## Description

### FIELD OF INVENTION

The present invention pertains to the field of long-term assist of the right ventricle of a heart to pump blood.

In particular, the invention relates to an implantable right ventricular assist device 1 configured to be implemented within the right ventricle of a patient's heart.

The invention also relates to a method to percutaneously, transluminally or transapically implant the Right Ventricular Assist Device 1 of the present invention within the right ventricle of a patient's heart.

The invention further relates to the use of the right ventricular assist device of the present invention to long-term assistance of the right ventricle of a patient's heart.

### BACKGROUND OF INVENTION

Congestive heart failure (CHF) is a progressive condition in which cardiac functions deteriorate over time. It is most common among people around 65 or older, but any one can be at risk. Heart failure includes everything from coronary artery disease, high blood pressure, and congenital heart defects to myocarditis, abnormal heart rhythms, valve disease, diabetes and obesity.

The term "failure" denotes the failure of the heart's muscular ventricle to pump enough blood out of the heart to meet the body's requirement, causing a backup, or "congestion", of blood in the veins and tissues. Over time, these conditions worsen, the heart beats more rapidly to compensate for its weakness, putting itself under great strain.

An estimated 26 million people worldwide currently suffer from CHF and roughly half of all people who develop CHF die within five years of diagnosis due to the limitations of current long-term treatment strategies.

While there is currently no cure for heart failure, options are available to treat its symptoms. Drug therapies, such as ACE inhibitors, vasodilators, diuretics, beta blockers or anticoagulants, Aldosterone inhibitor, neprilysin inhibitor, provide the foundation of heart failure treatment at any stage. When drugs are not efficient anymore, surgical treatments are taken into consideration and indicated depending on the source of the CHF. Among those treatments, the use of Ventricular Assist Device (VAD) has seen a growth in the last ten years.

VAD is a battery-operated, mechanical pump-alike device surgically implanted. It helps maintaining the pumping ability of a heart that can't effectively work on its own. VAD are generally used as "bridge to transplant", "bridge to recovery" or as a "destination therapy" in end-stage heart failure patients when heart transplantation isn't an option.

Today, the most used VAD are Left-sided Ventricular Assist Device (LVAD), and more particularly, are continuous flow type devices. The pump is implanted at the left ventricle then sends blood into the aorta. Due to high energy consumption, the pump is generally powered by an external battery, through a cable that exits the patient's body around the abdominal wall, the cable being linked to a control system. LVADs are often used for weeks to months and patients can be discharged from the hospital while waiting for a donor heart.

While most common heart failures are left-sided, it will lead to right-sided heart failures in 20-40% of the cases. However, in term of Right Assist Devices, few options are available. For example, several Bi-ventricular Assist Devices (BiVAD) have been developed, but are not used anymore due to the requirement of heavy surgery involving a low survival rate at six months (56%) and a high peri-operative adverse event rates (Survival after biventricular assist device implantation: An analysis of the Interagency Registry for Mechanically Assisted Circulatory Support Database; The Journal of Heart and Lung Transplantation, Vol 30, No 8, Aug. 2011).

Currently, the only solutions for right heart failure are either temporary VAD, heart transplant or full artificial heart. Temporary devices are linked to external power cables, which prevent patient's mobility. They can allow for an efficient hemodynamic recovery, but still present high hemorrhagic and thrombotic risks (40-50%).

EP 0156289 discloses for example, a right ventricular assist device for assisting the blood circulation, comprising a smooth seamfree sac formed of segmented polyurethane for receiving blood to be circulated, the sac having a single valveless passageway to provide a single inlet and outlet.

None of the prior art inventions sought to provide a permanently implantable blood pump that could be positioned in the cavity of the ventricle by means of less invasive surgical techniques without the need of cardiopulmonary bypass.

Thus, there is a need for an effective approach for the management of Right Heart Failure.

The goal of the present invention is to provide a Right Ventricular Assist Device (RVAD) percutaneously implanted within the right ventricle of a patient's heart for a definitive assistance, which does not require a heavy surgery and allows to address the major risks of VAD namely, hemorrhage and infection risks.

The RVAD of the invention relies on the heart native channels to assist the heart to transport the blood. The RVAD of the invention comprises an easily foldable continuous centrifugal flow pump; a fixation device and a power source.

Another object of the invention is to minimize the energy required to operate the device and in particular to transport the blood from the right ventricle to the pulmonary artery with the minimum energy and the shortest distance.

Yet another object of the invention is to avoid low or high shear stress that can occur when the blood comes into contact with the blades rotating at high RPM leading in the long term to thrombosis or hemolysis of the blood.

### SUMMARY

This invention thus relates to an implantable right ventricular assist device configured to be implanted within the apex or the admission chamber of the right ventricle of a patient's heart, in order to carry the patient's blood from the right ventricle to the pulmonary artery, said device comprising:
- a percutaneously deliverable centrifugal pump configured to carry the blood from the right ventricle to the pulmonary artery at a flow rate ranging from 1 to 10 L/min, said centrifugal pump comprising a body made of a foldable material, including: an inlet opening intended to let the blood enter inside de body; an outlet channel, intended to let the blood exit the body; a rotatable impeller displaying a thickness ranging from 2 mm to 10 mm, configured to carry the blood coming from the right ventricle, through said inlet opening, toward the pulmonary artery, through said outlet channel; a motor support ; and a motor connected to said rotatable impeller, said motor being configured to generate an electrical power ranging from 2 to 15 Watt, allowing said rotatable impeller to carry the blood from the right ventricle to the pulmonary artery with a rotation speed ranging from 3000 to 15000 rpm;
- a fixation device configured to secure said centrifugal pump within a portion of the pulmonary artery so as to carry the blood from the right ventricle to the pulmonary artery over a distance between 50 mm and 130 mm; and
- a power source connected to said motor and configured to delivers a voltage ranging from 2 to 15 Volt.

Such a configuration of the device is advantageous because it allows to minimize the energy to carry the blood from the right ventricle to the pulmonary artery. Also, by being placed directly in the right ventricle, the distance to transport the blood from the right ventricle to the pulmonary artery is minimized too.

According to one embodiment, said centrifugal pump delivers a pressure ranging from 15 to 75 mmHg.

According to one embodiment, said foldable material is chosen from PTFE, a Shape Memory Polymer or a Shape Memory alloy chosen from NiTinol, Copper-Zinc-Aluminum, Copper-Aluminum-Nickle, Iron-Manganese-Silicon or Copper-Aluminium-Manganes. This configuration is advantageous because it allows the centrifugal pump 2 to be implanted within the right ventricle via a catheter inserted into the patient's femoral artery or by high way via the subclavian or jugular vein or by a trans-apical minimally invasive incision under the costal.

According to one embodiment, said fixation device is a self-expanding stent configured to connect said outlet channel to a portion of the pulmonary artery.

According to one embodiment, the rotatable impeller comprises four blades.

According to one embodiment, said rotatable impeller is made of a foldable material.

According to one embodiment said rotatable impeller is made of a shaped memory alloy chosen from NiTinol, Copper-Zinc-Aluminum, Copper-Aluminum-Nickle, Iron-Manganese-Silicon or Copper-Aluminium-Manganese, preferably in NiTinol.

According to one embodiment, said rotatable impeller presents a shear rate ranging from 100 Pa to 300 Pa.

According to one embodiment, said motor delivers a torque value ranging from 1 to 11 mN.m.

According to one embodiment, said motor further comprises a gearbox configured to reduce the speed and increase the torque.

These configurations are advantageous because they allow the rotatable impeller to generate the desired flow rate and pressure to carry the blood from the right ventricle to the pulmonary artery without being harmful for the body and the blood by avoiding low shear rate or high shear rate which could lead in the long term to thrombosis or haemolysis of the blood.

According to one embodiment, said motor is an electric motor which receives electricity from said power source (4).

According to one embodiment, said motor is connected to said power source through a power wire.

According to one embodiment, said power source is a percutaneously implantable rechargeable battery.

According to one embodiment, the device of the invention further comprises an external controller configured to control said electric motor.

Advantageously, the communication between the external controller and the motor allows for an accurate control of the centrifugal pump depending on the patient's need.

According to one embodiment, the device of the invention further comprises a subcutaneously implantable sensor configured to communicate with said external controller.

In this configuration, the communication between the external controller and the subcutaneously implantable sensor allows to variate the pump speed either in synchronization with the heart rhythm, or the paced rhythm or out of synchronization with the heart rhythm or paced rhythm. Advantageously, it allows to adapt and optimize the blood flow depending on the patient's need.

### DEFINITIONS

In the present invention, the following terms have the following meanings:
- **"Centrifugal pump"** refers to a pump with the ability to transport fluids by the conversion of rotational kinetic energy to the hydrodynamic energy of the fluid flow. The rotational energy typically comes from an engine or electric motor. The fluid enters the pump impeller along or near to the rotating axis and is accelerated by the impeller, flowing radially outward into a diffuser or volute chamber (casing), from which it exits.
- **"Right Ventricle"** refers to the chamber within the heart that is responsible for pumping oxygen-depleted blood to the lungs. The right ventricle is one of the heart's four chambers. It is located in the lower right portion of the heart below the right atrium and opposite the left ventricle. As deoxygenated blood flows into the right atrium, it passes through the tricuspid valve and into the right ventricle, which pumps the blood up through the pulmonary valve and through the pulmonary artery to the lungs.
- **"Shape-Memory Alloy (SMA)":** refers to an alloy with the ability to change shape when one or more conditions are changed and then return back to its original shape on reversing those conditions. According to one embodiment, the SMA has the ability to change shape with the temperature. According to one embodiment, the SMA has the ability to change shape with the pressure. According to one embodiment, the shape-memory alloy is chosen from NiTinol, Copper-Zinc-Aluminum, Copper-Aluminum-Nickle, Iron-Manganese-Silicon or Copper-Aluminium-Manganese. According to a preferred embodiment, the shaped memory alloy is NiTinol.
- **"Shape-Memory Polymer (SMPS)"** refers to a polymer with the ability to change shape when one or more conditions are changed and then to return back to its original shape on reversing those conditions. According to one embodiment, the SMP has the ability to change shape with the temperature. According to one embodiment, the SMP has the ability to change shape with the pressure. According to one embodiment, the shape-memory polymer is chosen from Silicone resin, Polyurethane-based SMP, thermoset (covalently cross-linked) polymeric materials.
- **"Ventricular Assist Device (RVAD)"** refers to an implantable mechanical pump that helps pump blood from the lower chambers of a heart (the ventricles) to the rest of the body. A VAD is used in people who have weakened hearts or heart failures. According to one embodiment, the VAD is a left-sided VAD. According to one embodiment, the VAD is a right-sided VAD. According to one embodiment, the VAD is a bi-VAD.

### BRIEF DESCRIPTION OF THE DRAWINGS

**Figure 1** is a sectional view of a heart illustrating the location of the right ventricle assist device according to the invention within the right ventricle.
**Figure 2** is a sectional view of a heart illustrating the location of the right ventricle assist device according to the invention within the right ventricle.
**Figure 3A and B** are a combination of view. **Fig. 3A** is a front view of the centrifugal pump. **Fig. 3B** is a view from above of the centrifugal pump showing the rotatable impeller within the outlet part.
**Figure 4A, 4B and 4C** are a combination of view of the centrifugal pump 2. **Fig. 4A** is a view from above, **Fig. 4B** is a front view and **Fig. 4C** is a bottom view.
**Figure 5A, 5B and 5C** are a combination of view of the centrifugal pump 2 with an L-shape. **Fig. 5A** is an angled view. **Fig. 5B** is a view from above showing the rotatable impeller within the outlet part. **Fig. 5C** is a side view.

### DETAILED DESCRIPTION

The following detailed description will be better understood when read in conjunction with the drawings. For the purpose of illustration, the device is shown in the preferred embodiments. It should be understood, however, that the present invention is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

### Device

This invention relates to an implantable right ventricular assist device 1 configured to be implanted within the right ventricle of a patient's heart, comprising a percutaneously deliverable centrifugal pump 2 configured to carry the blood from the right ventricle to the pulmonary artery; a fixation device 3 configured to secure said centrifugal pump within a portion of the pulmonary artery so as to carry the blood from the right ventricle to the pulmonary artery; and a power source 4 connected to said motor.

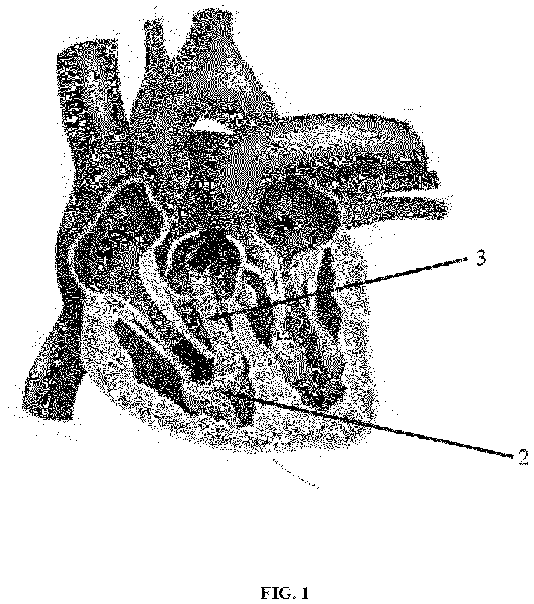

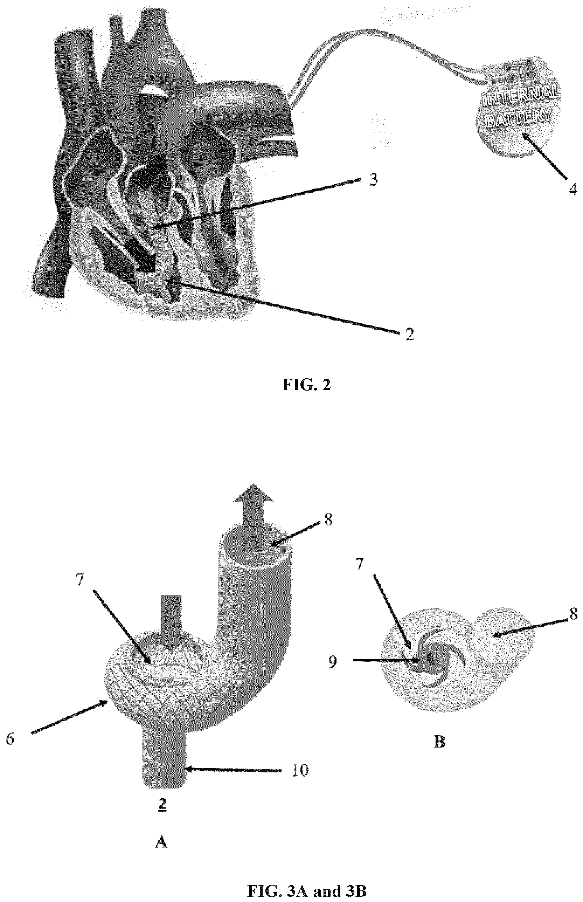

In figure 1 and figure 2, a right ventricular assist device 1 in accordance with the present invention is illustrated in conjunction with a patient's heart. As illustrated in figure 1, the right ventricular assist device 1 of the present invention includes a foldable centrifugal pump 2 which is percutaneously and transluminally delivered within the right ventricular apex of a patient and a transluminally deliverable fixation device 3. As illustrated in figure 1 and 2 the right ventricular assist device 1 is disposed within a portion of the right ventricle. In this embodiment, the foldable centrifugal pump 2, pumps blood downward from the superior vena cava and upward from the inferior vena cava. The blood is then sent upward through the pulmonary artery.

Advantageously, by being placed directly in the right ventricle, the energy to carry the blood to the pulmonary artery is minimized. The distance to transport the blood from the right ventricle to the pulmonary artery is minimized too.

According to one embodiment, the blood is carried from the right ventricle to the pulmonary artery over a distance between 50 mm and 130 mm.

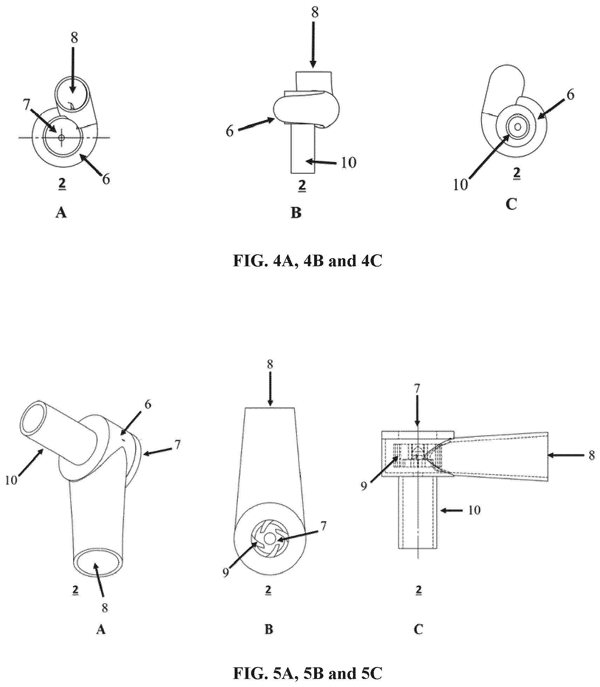

With reference to figure 3 and figure 4, the centrifugal pump 2 displays a general loopy shaping and comprises two openings 7 and 8 connected through a hollow body 6. Said body 6 includes therefore an inlet opening 7 and an outlet channel 8. The inlet opening 7 and outlet channel 8 are facing the same side of the body 6. The outlet channel 8 has two ends: a first end connected to the hollow body 6 and a second end intended to let the blood exit from the device 1. As can be grasped from figure 3A, the outlet channel 8 further displays a bend. The first end connects to the body 6 substantially perpendicularly to the blood inlet (through inlet opening 7) direction. After the centrifugal pump 2 has been put in right place within the heart's patient, the second end of the outlet channel 8 extends in a generally upwards direction, towards the patient's upper body. The blood exit direction is substantially parallel to the blood inlet direction. The blood coming from the right atrium enters the body 6 through the inlet opening 7 and leaves the body 6 towards the pulmonary artery through the outlet channel 8. Within the hollow body 6, the blood describes a loop: the entrance and exit directions of the blood are substantially parallel to each other while the entrance and exit flows circulate in opposite senses, as can be seen on figure 3A.

As illustrated in figure 5, the centrifugal pump 2 may displays an L-shape. In this embodiment the inlet opening 7 is perpendicular to the outlet channel 8. After the centrifugal pump 2 has been put in right place within the heart's patient, the outlet channel 8 extends in a generally upwards direction, towards the patient's upper body. The blood exit direction is substantially perpendicular to the blood inlet direction. The blood coming from the right atrium enters the body 6 through the inlet opening 7 and leaves the body 6 towards the pulmonary artery through the outlet channel 8. Within the hollow body 6, the blood describes an L-shape: the entrance and exit directions of the blood are substantially perpendicular to each other.

As illustrated in figure 1 and 2, the fixation device 3 may be a self-expending stent connected to the outlet channel 8, configured to secure, or anchor, the foldable centrifugal pump 2 within a portion of the pulmonary artery, for long-term use, to assist the pumping of blood from the right ventricle upwardly through the pulmonary artery.

The hollow body 6 also comprises a rotatable impeller 9. Said impeller 9 is configured to carry the blood coming from the right atrium; through the inlet opening 7, toward the pulmonary artery, through the outlet channel 8. As shown on figure 3B, the rotatable impeller 9 is a four blades impeller. The impeller 9 may also be a two or three blades impeller.

The rotatable impeller 9 further presents a thickness ranging from 2 mm to 10 mm, preferably ranging from 4 mm to 8 mm, and a diameter ranging from 5 mm to 20 mm, preferably ranging from 10 mm to 20 mm.

According to the embodiment illustrated on figures 3 and 4, said body 6 presents a length ranging from 30 mm to 130 mm and a diameter ranging from 15 mm to 30 mm. The inlet opening 7 presents a diameter ranging from 5 mm to 20 mm. The outlet 8 presents a diameter ranging from 5 mm to 20 mm.

As illustrated in figure 3B, the rotatable impeller 9 is disposed within the inlet opening 7. The impeller 9 is preferably powered by an electric motor (not shown), in communication with said rotatable impeller 9. Said motor is disposed inside a motor support 10, situated below the impeller 9, and is configured to generate the required power and torque for said rotatable impeller 9 to carry the blood from the right ventricle to the pulmonary artery with the desired flow rate and pressure.

According to the described embodiment the motor delivers an electrical power ranging from 2 to 15 Watt, preferably ranging from 3 to 7 Watt.

The motor also delivers a mechanical power ranging from 2 to 15 Watt, preferably ranging from 3 to 7 Watt.

According to said embodiment, the motor delivers a torque value ranging from 1 to 11 mN.m, preferably ranging from 3 to 8 mN.m.

The mechanical power is directly linked to the torque value generated by the motor. Power and torque value allow the rotatable impeller 9 to generate the desired flow rate and pressure to carry the blood from the right ventricle to the pulmonary artery. The motor also delivers a rotation speed ranging from 3000 to 15000 rpm, preferably ranging from 8000 to 12000 rpm.

The motor may further comprise a gearbox. The gearbox allows, if necessary, to reduce the speed and increase the torque, in order to reach the desired flow rate and pressure of 5 liters/minute and 25 mmHg respectively.

When well put in place inside the patient's heart and activated, the impeller 9 of the centrifugal pump 2 allows to generate the adequate pressure at the outlet 8 for the blood to rise and reach the pulmonary artery, after the blood passes through the inlet opening 7. Said adequate pressure is ranging from 15 to 75 mmHg, preferably 25 mmHg.

The rotation of the impeller 9 generates a velocity ranging from 0,2 m/s to 1 m/s at the outlet channel 8 of the centrifugal pump 2. The generated velocity is preferably about of 0,6 m/s.

The rotatable impeller 9 further presents a shear rate ranging from 200 Pa to 350 Pa Low shear rate or high shear rate that may occur in the long term and be harmful for the blood by leading to thrombosis or haemolysis of the blood.

The velocity generated at the outlet channel 8 of the centrifugal pump 2 is enough to generate the desired flow rate to carry the blood from the right ventricle to the pulmonary artery.

Desired flow rate is ranging from 0.5 liters/minute to as high as 10 liters/minute, preferably 5 liters/minute depending upon the needs of the patient.

According to one embodiment, the body 6 of the centrifugal pump 2 is made of bio-compatible materials, including plastic materials, having the requisite strength and bio-compatibility characteristics which permit the desired use in a patient's body.

According to a preferred embodiment, the body 6 of the centrifugal pump 2 is made of a suitable foldable material. According to one embodiment, the body of the centrifugal pump 2 is made of PTFE. According to a preferred embodiment, the body 6 of the centrifugal pump 2 is made of a Shape Memory Polymer (SMP), such as Silicone resin, Polyurethane-based SMP, thermoset (covalently cross-linked) polymeric materials. According to a more preferred embodiment, the body 6 of the centrifugal pump 2 is made of Shape Memory Alloy (SMA). According to one embodiment, the Shape Memory Alloy is chosen from NiTinol, Copper-Zinc-Aluminum, Copper-Aluminum-Nickle, Iron-Manganese-Silicon or Copper-Aluminium-Manganese. According to a preferred embodiment, the Shape Memory Alloy is NiTinol.

Advantageously, these embodiments allow the centrifugal pump 2 to be implanted within the right ventricle via a catheter inserted into the patient's femoral artery. The implantation method will be described further.

According to one embodiment, as illustrated in figure 2, the power is supplied to the motor of the centrifugal pump 2 by a power source 4.

According to one embodiment, the power source 4 is a subcutaneously implantable battery. which may be implanted in the pectoral area of the patient or in a pocket made in the abdomen of the patient.

According to one embodiment, the subcutaneously implantable battery 4 is a rechargeable battery and may be rechargeable per induction.

According to the embodiment illustrated in figure 2, a power wire 5 is associated with the electric motor. The power wire 5 extends from the electric motor to the power source 4. According to this embodiment, the power wire 5 passes through the superior vena cava towards the pectoral area where it may be connected to the power source 4.

In the described embodiment, the power source 4 delivers a voltage ranging from 2 to 15 Volt, preferably ranging from 4 to 8 Volt.

According to one embodiment, the rotatable impeller 9 is made of bio-compatible materials, including plastic materials, having the requisite strength and bio-compatibility characteristics which permit the desired use in a patient's body.

According to one embodiment, the rotatable impeller 9 is made of suitable foldable material. According to one embodiment, the rotatable impeller 9 is made of PTFE. According to one embodiment, the body of the rotatable impeller 9 is made of a Shape Memory Polymer, such as Silicone resin, Polyurethane-based SMP, thermoset (covalently cross-linked) polymeric materials. According to one embodiment, the body of the rotatable impeller 9 is made of Shape Memory Alloy. According to one embodiment, the Shape Memory Alloy is chosen from NiTinol, Copper-Zinc-Aluminum, Copper-Aluminum-Nickle, Iron-Manganese-Silicon or Copper-Aluminium-Manganese. According to a preferred embodiment, the shaped memory alloy is NiTinol.

Advantageously, in these embodiments, the rotatable impeller 9 allows to generate the desired flow rate and pressure to carry the blood from the right ventricle to the pulmonary artery without being harmful for the body and by avoiding low shear rate or high shear rate which could lead in the long term to thrombosis or haemolysis of the blood.

According to one embodiment, the right ventricular assist device 1 further comprises an external controller configured to control the electric motor destined to communicate with the motor and to provide information to the patient implanted with the device 1. The external controller communicates, in a well know way, with the motor via a cable or a wireless protocol. The external controller may for example be a portable device such as a smartphone, tablet or personal computer. According to one embodiment, the external controller is equipped with a Bluetooth chip or a with a WIFI connection system. Said external controller could be worn on the patient's belt or up on a holster-type system, or strapped to the patient's leg via a Yelcro® type attachment means, or other suitable attachment structure.

Advantageously, the communication between the external controller and the motor allows for an accurate control of the centrifugal pump 2 depending on the patient's need.

According to the current described embodiment, the right ventricular assist device 1 further comprises a subcutaneously implantable sensor configured to communicate with the external controller.

The communication between the external controller and the subcutaneously implantable sensor allows an accurate control of the centrifugal pump 2 depending on the patient's need.

The communication between the external controller and the subcutaneously implantable sensor allows to variate the pump speed either in synchronization with the heart rhythm, or the paced rhythm or out of synchronization with the heart rhythm or paced rhythm. Advantageously, it adapts and optimizes the blood flow depending on the patient's need.

The subcutaneously implantable sensor may also have the ability to sense the native electrocardiogram of the patient or the paced rhythm. In this case, the sensor communicates the measured data to the external controller, which may vary the pump speed accordingly.

According to another embodiment, the external controller is able to communicate directly or indirectly with an implanted pacemaker or defibrillator device, to optimize flow depending on the patient's need. The sensor may be able to sense when the patient is supine or lying down and communicates the information to the external controller which may decrease or increase overall pump speed to compensate for decreased need while supine.

The sensor may also be able to sense other physiologic parameters such as bioimpedance, body motion or cardiac performance parameters and communicates the information to the external controller which may adjust pump speed to optimize flow of blood to the body.

### Method

The invention also relates to a method and procedure to percutaneously and transluminally implant the Right Ventricular Assist Device 1 of the present invention within the right ventricle of a patient's heart. The method may aim at providing long-term assistance to the right ventricle of a heart or aim at providing short-term assistance on a transitional basis, for example for a patient waiting for a heart implant.

Said method comprises the steps of:
1) inserting the already folded centrifugal pump 2 and its associated motor into a self-expanding stent;
2) inserting a catheter up to the patient's pulmonary artery;
3) inserting the stented centrifugal pump 2 and its associated motor into the catheter;
4) delivering the stented centrifugal pump 2 and its associated motor to the pulmonary artery;
5) securing the outlet channel 8 within a portion of the pulmonary artery thanks to the self-expanding stent;
6) removing the catheter; and
7) securing the centrifugal pump 2 and its associated motor within the right ventricle of the patient's heart.

According to one embodiment, the catheter is inserted up to the pulmonary artery via the femoral artery or by high way via the subclavian or jugular vein or by a trans-apical minimally invasive incision under the costal.

According to one embodiment, the method to percutaneously and transluminally implant the Right Ventricular Assist Device 1 further comprises a step of associating a first end of a power wire with the motor and a second end of a power wire with a power source 4.

According to one embodiment, the method to percutaneously and transluminally implant the Right Ventricular Assist Device 1 further comprises a step of subcutaneously implanting the power source in the pectoral area of the patient or in a pocket made in the abdomen of the patient.

According to one embodiment, the centrifugal pump 2 and its associated motor are attached within the right ventricular apex. According to one embodiment, the centrifugal pump 2 and its associated motor are attached within the admission chamber of the right ventricle. In these embodiments, the centrifugal pump 2, pumps blood downward from superior vena cava and upward from the inferior vena cava, the blood is then sent upward through the pulmonary artery.

According to one embodiment, the method to percutaneously and transluminally implant the Right Ventricular Assist Device 1 further comprise a step of controlling the pump speed either in synchronization with a rhythm of the heart or out of synchronization with the rhythm of the heart.

### Use

The invention also relates to the use of the right ventricular assist device of the present invention for long-term assist of the right ventricle of a patient's heart to pump blood.

According to one embodiment, the right ventricular assist device of the present invention is used in right-sided heart failure.

According to one embodiment, the right ventricular assist device of the present invention is used in congestive heart failure.

While various embodiments have been described and illustrated, the detailed description is not to be construed as being limited hereto. Various modifications can be made to the embodiments by those skilled in the art without departing from the true spirit and scope of the disclosure as defined by the claims.

## Claims

1. An implantable right ventricular assist device (1) configured to be implanted within the apex or the admission chamber of the right ventricle of a patient's heart, in order to carry the patient's blood from the right ventricle to the pulmonary artery, said device (1) comprising:
a percutaneously deliverable centrifugal pump (2) configured to carry the blood from the right ventricle to the pulmonary artery at a flow rate ranging from 1 to 10 L/min, said centrifugal pump (2) comprising:
- a body 6 made of a foldable material, including:
an inlet opening (7) intended to let the blood enter inside de body (6);
- an outlet channel (8), intended to let the blood exit the body (6);
- a rotatable impeller (9) displaying a thickness ranging from 2 mm to 10 mm, configured to carry the blood coming from the right ventricle, through said inlet opening (7), toward the pulmonary artery, through said outlet channel (8);
- a motor support 10; and
- a motor connected to said rotatable impeller (9), said motor being configured to generate an electrical power ranging from 2 to 15 Watt, allowing said rotatable impeller (9) to carry the blood from the right ventricle to the pulmonary artery with a rotation speed ranging from 3000 to 15000 rpm;
a fixation device (3) configured to secure said centrifugal pump (2) within a portion of the pulmonary artery so as to carry the blood from the right ventricle to the pulmonary artery over a distance between 50 mm and 130 mm; and
a power source (4) connected to said motor and configured to delivers a voltage ranging from 2 to 15 Volt.

2. The right ventricular assist device (1) according to claim **1,** wherein said centrifugal pump (2) delivers a pressure ranging from 15 to 75 mmHg.

3. The right ventricular assist device (1) according to claim **1** or claim **2,** wherein said foldable material is chosen from PTFE, a Shape Memory Polymer or a Shape Memory alloy chosen from NiTinol, Copper-Zinc-Aluminum, Copper-Aluminum-Nickle, Iron-Manganese-Silicon or Copper-Aluminium-Manganes.

4. The right ventricular assist device (1) according to any one of claim **1** to **3,** wherein said fixation device (3) is a self-expanding stent configured to connect said outlet channel (8) to a portion of the pulmonary artery.

5. The right ventricular assist device (1) according to any one of claim **1** to **4,** wherein said rotatable impeller (9) comprises four blades.

6. The right ventricular assist device (1) according to claim **5,** wherein said rotatable impeller (9) is made of a foldable material.

7. The right ventricular assist device (1) according to claim **6,** wherein said rotatable impeller (9) is made of a shaped memory alloy chosen from NiTinol, Copper-Zinc-Aluminum, Copper-Aluminum-Nickle, Iron-Manganese-Silicon or Copper-Aluminium-Manganese, preferably in NiTinol.

8. The right ventricular assist device (1) according to any one of claims **1** to **7,** wherein said rotatable impeller (9) presents a shear rate ranging from 100 Pa to 300 Pa.

9. The right ventricular assist device (1) according to any one of claim **1** to **8,** wherein said motor delivers a torque value ranging from 1 to 11 mN.m.

10. The right ventricular assist device (1) according to any one of claim **1** to **9,** wherein said motor further comprises a gearbox configured to reduce the speed and increase the torque.

11. The right ventricular assist device (1) according to any one of claim **1** to **10,** wherein said motor is an electric motor which receives electricity from said power source (4).

12. The right ventricular assist device (1) according to claim **11,** wherein said motor is connected to said power source (4) through a power wire (5).

13. The right ventricular assist device (1) according to any one of claim **1** to **12,** wherein said power source (4) is a percutaneously implantable rechargeable battery.

14. The right ventricular assist device (1) according to any one of claim **1** to **13,** further comprising an external controller configured to control said electric motor.

15. The right ventricular assist device (1) according to any one of claim **1** to **14,** further comprising a subcutaneously implantable sensor configured to communicate with said external controller.
